# EUROPEAN PATENT APPLICATION

(11) **EP 1 145 694 A1**
(43) Date of publication of application: **17.10.2001**
(21) Application number: 00870066.8
(22) Date of filing: 12.04.2000
(51) Int. Cl.: A61F 5/48

(54) **Disposable covering sheet assemblies**

(71) Applicant: The Procter & Gamble Company, Cincinnati, Ohio 45202 (US)
(72) Inventor: Dauger-Strauss, Corinne, 1040 Brussels (BE); Russo, Elisabetta Laura, 00142 Rome (IT)
(74) Representative: Canonici, Jean-Jacques

(57) **Abstract**

The present invention provides disposable covering sheets assemblies comprising a stack of individual covering sheets, said individual covering sheets being joined in stacked relationship by appropriate means, said means allowing the lifting and removal of individual sheets to expose the underlying sheet, characterized in that each sheet has a moisture vapour transport rate of at least 50g/m².24h. The assemblies of the present invention are preferably used in disposable bed or pillow covers.

The present invention is also directed to disposable pillow cases including the assemblies herein.

## Description

### FIELD OF THE INVENTION

The present invention relates to disposable covering sheet assemblies comprising a stack of peelable individual covering sheets, which are removed after use to expose the underlying sheet; the covering sheet assemblies of the present invention are particularly useful for use in disposable bed sheets and pillow cases;

### BACKGROUND OF THE INVENTION

Disposable covering sheets, in particular for bedding articles, are well known in the art; such articles include mattress covers, bed sheets, pillow liners and pillow cases; the function of such covers, in particular when used in a hospital or institution context, is often to protect the underlying part of the bed, such as the mattress, in which case the covers are impermeable and preferably absorbent; such disposable covers for mattresses can also be used to provide anti-allergy benefits, by providing a barrier against dust mites.
Disposable bed sheets and pillow cases can also known as single-use alternatives to woven articles in e.g. the transportation industry.
Also known are disposable lining sheets for mattresses or pillows which are coated or impregnated with aromas or volatile therapeutic materials.
However, the above articles are designed for single use and need to be replaced in their entirety when they have been utilized and are ready for disposal.
US 3 761 973 discloses plastic back absorption pad sheets assemblies in the form of a stack of individual pad sheets, used to absorb moisture and other bodily discharges; the soiled sheets can be readily removed to expose a clean absorption pad sheet; each pad sheet has a plastic back sheet and is therefore moisture and vapour impermeable.
There is a need to provide consumer or patients with multi-use disposable covers, in particular for beds or pillows, providing renewed freshness/confort/cleanliness benefits to the user by offering the possibility to readily remove individual sheets and expose the underlying fresh sheet;
For such a use, and particularly as bed pillows covers are in direct contact with the body, it is essential that each such cover be breathable, i.e. that they allow the passage of moisture vapour.

It is a particular object of the present invention to provide disposable bedding articles, in particular bed sheets and particularly pillow cases, which can provide the user with a renewed freshness/cleanliness sensation at the time of going to bed, thus allowing to create a relaxing atmosphere which in times influences the readiness to fall asleep and the quality of sleep.

It is also an object of the present invention to provide disposable bedding articles such as bed sheets and pillow cases, which combine barrier and protection properties, for domestic or institutional usage together with a renewed freshness/cleanliness sensation.

### SUMMARY OF THE INVENTION

The present invention provides disposable covering sheets assemblies comprising a stack of individual covering sheets, said individual covering sheets being joined in stacked relationship by appropriate means, said means allowing the lifting and removal of individual sheets to expose the underlying sheet, characterized in that each sheet has a moisture vapour transport rate of at least 50g/m².24h. The assemblies of the present invention are preferably used in disposable bed or pillow covers.

The present invention is also directed to disposable pillow cases including the assemblies herein.

### DETAILED DESCRIPTION

The present invention relates to multi-use disposable covering articles in the form of an assembly of individual covering sheets; by multi-use, it is meant that each single article allows for more than one utilization as fresh covering, as the individual sheets can be removed after use to readily expose the fresh sheet underneath;
The assemblies of the invention are in the form of stacks or individual sheets, which are joined in their stacked relationship by appropriate means, said means allowing the lifting and removal of individual sheets to expose the underlying sheet;

Any appropriate means for keeping the individual covering sheets in their stacked relationship, and allowing the lifting and removal of individual sheets to expose the underlying sheet, can be used :
For example, the individual sheets can be permanently joined in their stacked relationship along at least one edge, preferably along two parallel edges and have perforations extending laterally throughout the sheet in a direction parallel to said edge or edges, and at a distance from said edge or edges, each of the individual sheets thereby being separable for permitting the easy lifting or removal of one of the sheets of the stack from the other; the permanent joining as said at least one edge can be achieved by e.g. mechanical means such as sewing or stapling, adhesive means, or heat-sealing means, either directly between each individual sheet, or via a separate material holding the sheets together;

Alternatively, the individual sheets can be releasably joined to each other through mechanical means which e.g. hold the individual sheets together without providing a fixed link to the sheets, or via releasable-adhesive means extending through a part or the entirety of their surface; such temporary adhesive means may be in the form of strips or bands preferably positioned along at least two parallel edges; pressure sensitive adhesive composition can also be coated on part of the totality of the individual sheets:
Combination of permanent and reasable means as described above are also encompassed.
Each individual sheet must be made of a material which is moisture vapour permeable, the moisture vapour transfer rate being at least 50g/m².24h, preferred at least 300g/m².24h, most preferably or least 500g/m².24h; the moisture vapour transfer rate (MVTR) is measured at 23°C using the ASTM E-96 "Upright Cup" method.
Moisture vapour permeable materials suitable as individual sheets herein include, but are not limited to: fibres, fibrous batts, non-wovens, wovens, papers, micro-porous or porous membranes, films such as polymeric films, perforated or apertured films, macroscopically expanded films, cloth, etc.

Preferred materials are nonwovens, including nonwovens with stretchable properties; the nonwoven can be selected from any well known material having appropriate tensile strength characteristic; the nature and caliper of the nonwoven will also be chosen according to the absorbent characteristics desired; the nonowoven can also be chosen such that it it has biodegradability properties.

The individual sheets herein may comprise any suitable material or structure which is capable of providing a liquid barrier, and preferably also a barrier against dust mites and related allergens and microorganisms, in addition to providing moisture vapour permeability.

Each individual sheet of the assemblies herein may have such a barrier function; alternatively only a limited number of sheet may have such a barrier function, and the assemblies herein may contain only one of such individual sheets having a barrier function, especially as the lowermost sheet of the assembly.

Such structures or materials capable of providing liquid barrier and moisture vapour permeably can comprise a single layer, or multiple layers, typically of different materials, laminated together to form a composite. An example are structures comprising thermoplastic microporous films, e.g. laminated to fibrous layers such as nonwoven layers.
Particularly preferred materials are composite structures with e.g. nonwoven as a substitute containing a hydrophilic continuous films, also known as "monolithic films', that do not allow the flow of moisture vapour through open pores or apertures in the material, but do transfer substantial amounts of moisture vapour through the film by absorbing water on one side of the film where the moisture vapour concentration is higher, and absorbing or evaporating it on the opposite side of the film where the moisture vapour concentration is lower.
Especially preferred materials for providing barrier properties as well as moisture vapour transfer are thermoplastic compositions described in 0 963 760 and0 964 026, and laminates comprising said thermoplastic compositions, also described therein.

If the individual sheets are made of the laminates described above, the adhesivity between sheets can be advantageously provided by the selection of the tackifying resin; indeed the thermoplastic composition described in the above application typically include resins or blends of tackifying resins. Preferred resins, which may be present by up to 50% by weight of the thermoplastic composition, may be selected from rosins and rosin esters, hydrocarbon resins, aliphatic resins, terpene and terpene-phenolic resins, aromatic resins, synthetic C₅ resins, mixtures of synthetic C₅-C₉ resins, and mixtures thereof;
By suitably selecting the tackifier resin or blend of tackifier resins, it is possible to adjust the residual tackiness at room temperature of the thermoplastic composition, and therefore of the layer formed from said thermoplastic composition, to the extent that it has the typical characteristics of a pressure sensitive adhesive.

Preferred tackifier resins, or blend of tackifier resins still have a softening point of 125°C or less, and are selected from the group consisting of rosin and rosin esters, terpene-Phenolic resins, aromatic resins, and mixtures thereof.

More preferably, the thermoplastic compositions according to the above mentioned applications comprise a blend of tackifier resins, selected as described above, wherein moreover from 0% to 20%, preferably from 2% to 15%, by weight of said blend of tackifier resins comprises a resin or blend of resins having a softening point of less than 25°C, and from 80% to 100%, preferably from 85% to 98%, by weight of said blend of tackifier resins comprises a resin or blend of resins having a softening point of at least 70°C.

The assemblies of the present invention will provide through their inherent design, a freshness benefit and impression to the user, thanks to the renewbility of the individual sheets; it may however be desirable to enhance the fresheness impression and the relaxation benefit by coating or impregnating the individual sheets with materials such as perfumes, which will release a fragrance during their utilisation; preferably achieving a slow and gradual release over time.

The individual sheets herein may also be coated or impregnated with other materials selected from insecticides, deodorants, odor control materials, moisture absorbents, volatile therapeutic materials, aromatic oils and mixtures thereof.

Odor control materials include odor absorbent/encapsulation agents, and odor-prevention agents, selected from materials known for this purpose.

Moisture absorbent materials which may be incorporated include super absorbent materials of the type used in disposable diapers. Lower concentrations may be incorporated where the cover sheet, is simply to be used to absorb perspiration in hot conditions.

Volatile therapeutic materials include materials which assist in keeping nasal passages clear of congestion in cold or allergy sufferers, for example menthol, camphor, eucalyptus oil and similar oil, as well as other materials which can benefit a patient by absorption through the nasal passages and the lungs as a result of prolonged exposure. The therapeutic materials may include extracts of herbs and the like which help the user to relax or to sleep. Aromatic oils, such as those referred to as 'aromatherapy oils' may also be impregnated into the substrate.

The disposable covering assemblies of the present invention can be used in a variety of fields for both domestic, industrial or institutional usage; such usages include, but are not limited to, multi-use coverings for headrests and seats, both for domestic use or institutional use, and for use in the transportation industry, multi-use table covers of dining sets, and multi-use coverings for medical examination tables;
The preferred utilization of the assemblies herein is in bedding articles, both for domestic and institutional usage; The assemblies of the present invention are particularly useful for use in disposable bed sheets and pillow cases.

### DISPOSABLE MULTI-USE PILLOW CASES

The preferred embodiment herein is for a disposable multi-use pillow case; as the pillow is the bedding article which is in contact with nose and mouth of the user, the renewed fresheness benefit that is brought by the assemblies of the present invention will be particularly relevant, and the related benefits provided to the user consequently improved.

A pillow case according to the present invention includes at least one assembly according to the present invention preferably containing from 2 to 20 sheets, most preferred from 3 to 10 sheets;

The pillow cases herein may be constructed such that both of their sides include an assembly according to the present invention, or such that one of their sides include or is made of such an assembly;

The pillow cases herein can be made in any shape and dimension as desired, and typically have an opening for inserting and removing the pillow.

The individual sheets can be decorated with printed designs, which can be different on each sheet of the stack and be arranged in a sequence for e.g. entertainment or educational purposes for children.

### BRIEF DESCRIPTION OF THE DRAWINGS

The drawings herein are intented to illustrate preferred embodiments of the present invention, but are not meant to limit in any way to limit its scope.
Figure 1 is a partial and simplified view of an assembly according to the present invention.
Figure 2 is a perspective and simplified view of a pillow case according to the present invention.
Figure 3 is a sectional view of the pillow case of Figure 2.

### DETAILED DESCRIPTION OF THE DRAWINGS

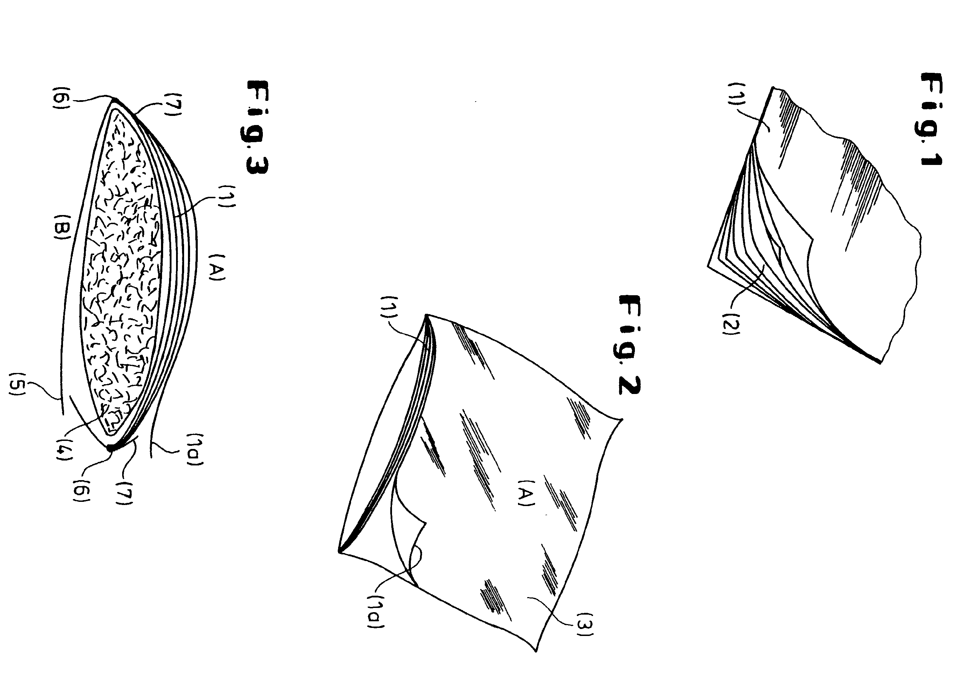

Referring to Figure 1, the assembly (1) comprises a stack of individual sheets (2) which are peelable, i.e. removable from each other to expose the underlying sheet.

Figure 2 shows a filled pillow case (3), which uses an assembly (1) as one of its surface (A), and shows the top individual sheet (1a) partially peeled away.

Figure 3 shows a section of the pillow case of Figure 2, showing both surfaces (A) and (B) of the pillow case, a pillow (4) inserted in the pillow case, and an opening (5) to insert and remove the pillow.
The assembly (1) is used as surface (A) of the pillow, which serves as user-facing side; the top individual sheet (1a) is showed as partially peeled away.
The individual sheets (1) are kept together in their stacked relationship by being heat sealed together at joints (6); joints (6) also constitute the joining point with surface (B) of the pillow case; perforations (7) are present at a distance from both joints and parallel to the joints to allow the sheets to be detached and peeled away.

## Claims

1. A disposable covering sheet assembly, comprising a stack of individual covering sheets, said individual covering sheets being joined in their stacked relationship by appropriate means, said means allowing the lifting and removal of individual sheets to expose the underlying sheet, **characterized in that** each sheet has a moisture vapour transport rate of at least 50g/m².24h.

2. A disposable covering sheet assembly according to claim 1, wherein said moisture vapour transport rate is if at least 300g/m².24h, preferably at least 500g/m².24h.

3. A disposable covering sheet assembly according to claims 1-2, wherein said individual sheets are joined in their stacked relationship by permanent means, releasable means, or combinations thereof.

4. A disposable covering sheet assembly according to claims 1-3, wherein said individual sheets are made of a nonwoven.

5. A disposable covering sheet assembly according to claims 1-4 is capable of providing liquid barrier characteristics for liquids and preferably for dust mites and related allergens and microorganisms.

6. A disposable covering sheet assembly according to claim 5, wherein each individual sheet is capable of providing such barrier characteristics.

7. A disposable covering sheet assembly according to claims 1-6, wherein each individual sheet is coated or impregnated with a material selected from perfumes, insecticides, deodorants, odor control agents, moisture absorbents, volatile therapeutic materials, aromatic oils, and mixtures thereof.

8. A disposable pillow case containing an assembly according to claims 1-7.

9. A disposable pillow case according to claim 8, wherein said assembly constitutes one side of said pillow case.

10. A disposable pillow case according to claims 8-9, wherein said assembly contains 2 - 20, preferably 3 - 10 individual sheets.
